# EUROPÄISCHE PATENTSCHRIFT

(11) **EP 0 592 883 B1**
(45) Veröffentlichungstag und Bekanntmachung des Hinweises auf die Patenterteilung: **01.05.1996**
(21) Anmeldenummer: 93115822.4
(22) Anmeldetag: 30.09.1993
(51) Int. Cl.: B01D 61/36, C07C 29/76, C07C 31/04, C07C 31/10, C07C 68/08, C07C 69/96

(54) **Verfahren zur Abtrennung von Alkanolen, Alkohol/Wasser-Gemischen oder Wasser von sauerstoffhaltigen organischen Verbindungen**
Method of separating alcohols, alcohol/water mixture or water from oxygen containing organic compound
Procédé de séparation d'alcools, de mélanges alcool-eau ou d'eau à partirde composés organiques renfermant

(30) Priorität: 13.10.1992 DE 4234525
(43) Veröffentlichungstag der Anmeldung: 20.04.1994
(73) Patentinhaber: BAYER AG, D-51368 Leverkusen (DE)
(72) Erfinder: Nickel, Andreas, Dipl.-Ing., D-58300 Wetter (DE); Arlt, Wolfgang, Prof. Dr., c/o TU Berlin, D-10623 Berlin (DE); Janisch, Ingo, Dr., D-51515 Kürten (DE); Wagner, Paul, Dr., D-40597 Düsseldorf (DE); Klausener, Alexander, Dr., D-50670 Köln (DE)

(56) Entgegenhaltungen:
- EP-A- 0 476 370
- US-A- 4 798 674
- US-A- 4 960 519
- US-A- 5 151 190

## Beschreibung

Die vorliegende Erfindung betrifft ein Verfahren zur Abtrennung eines ersten Alkanols mit 1 bis 3 C-Atomen oder eines Gemisches dieses ersten Alkanols mit Wasser von sauerstoffhaltigen organischen Verbindungen mit 3 bis 7 C-Atomen, bestehend aus einem Zweiten Alkanol und/oder Dialkylcarbonat, wobei das erste Alkanol stets mindestens 2-C-Atome weniger hat als jede der sauerstoffhaltigen organischen Verbindungen oder zur Abtrennung von Wasser von aus Dialkylcarbonat bestehenden sauerstoffhaltigen organischen Verbindungen mit 3 bis 7 C-Atomen. Die Abtrennung erfolgt durch Pervaporation oder Dampfpermeation an einer Membran, die durch Plasmapolymerisation hergestellt worden ist.

Das erfindungsgemäße Verfahren ermöglicht vereinfachte Trennungen, insbesondere in solchen Fällen, in denen durch Vorliegen eines Azeotrops destillative Trennverfahren versagen. Dies spielt für viele Gemische verschiedener Alkanole, insbesondere mit einem zusätzlichen Gehalt an Wasser, als auch für die Trennung von Dialkylcarbonaten von den zugrundeliegenden Alkoholen, wiederum auch in solchen Fällen, in denen ein Gehalt an Wasser gegeben ist, oder für die Abtrennung von Wasser allein, beispielsweise von Dialkylcarbonaten, eine bedeutende Rolle. Ein sehr wichtiges technisches Problem dieser Art ist die Abtrennung von Methanol aus Dimethylcarbonat, gegebenenfalls in Gegenwart von im Gemisch vorhandenem Wasser. Ein weiteres wichtiges Problem dieser Art ist die Abtrennung von Wasser von Dimethylcarbonat.

Die Trennung der beschriebenen Gemische erfolgte bisher beispielsweise durch Zweidruckdestillation (vgl. DE-A-2 607 003 und JP 02-212 456), da es bekannt ist, daß die Zusammensetzung von azeotropen Gemischen druckabhängig ist. Vielfach reicht die hierbei erhaltene Trennrate nicht aus und muß ergänzt werden durch andere physikalische Prozesse, beispielsweise durch eine Kristallisation. Zudem verursachen Druckapparaturen stets höhere Investitionskosten; wegen des durch den Druck erhöhten Temperaturniveaus ist stets auch mit einer erhöhten Bildung von Nebenprodukten zu rechnen.

Neben der Destillation unter erhöhtem Druck wurde auch versucht, Gemische der beschriebenen Art durch eine Extraktivdestillation zu trennen. Im Falle der Auftrennung des Azeotrops Dimethylcarbonat/Methanol wird dabei bevorzugt Wasser als Extraktionsmittel verwendet (vgl. DE-A-2 450 856). Das benötigte Verhältnis von Wasser zu Dimethylcarbonat liegt bei 20:1. Diese große Wassermenge muß in einer weiteren Destillationskolonne wieder vom Methanol getrennt werden. Hierbei ist es zusätzlich nachteilig, daß Wasser im Gegensatz zu organischen Verbindungen eine mehr als 4 mal so hohe Verdampfungswärme und eine mehr als doppelt so hohe Wärmekapazität hat; beides führt zu einem erhöhten Energieverbrauch.

Auch eine Verbesserung der Extraktivdestillation durch Verwendung von organischen Lösungsmitteln als Zusatzstoffen ist gleichermaßen nachteilig durch die Notwendigkeit, diese Zusatzstoffe aufzuarbeiten und zu recyclisieren (vgl. EP-A-00 1780; DE-A-2 706 684 und DE-A-2 737 265).

Es ist daher bereits versucht worden, schwierig zu trennende Gemische mit Hilfe der Membrantechnologie aufzutrennen. So beschreibt EP-A-331 846 die Trennung kurzkettiger Alkohole von sauerstoffhaltigen organischen Verbindungen, wie Ethern, Aldehyden, Ketonen oder Estern, mit Hilfe einer Mehrschichtmembran. Die Trennmembran dieser Anordnung besteht entweder aus einer Polyvinylalkohol-Schicht, die mit aliphatischen Polyaldehyden vernetzt ist, oder aus einem Harz, das auch bei Ionenaustauschern eingesetzt wird und Säuregruppen enthält, die mit quaternären Ammoniumsalzen modifiziert wurden. Als Trägermaterial für die Mehrschichtmembran wird ein Polyestergewebe verwendet; eine weiterhin benutzte poröse Stützmembran besteht aus einem Polysulfongewebe. Diese Membran ist relativ kompliziert aufgebaut. Infolge ihres chemischen Aufbaus ist die Arbeitstemperatur auf 70°C begrenzt und ergibt hierbei einen niedrigen maximalen Fluß von 1,5 kg/m²·h. Dies setzt dem technischen Einsatz Grenzen. Die beschriebenen Permeatanreicherungen an Methanol von 73 % auf lediglich 93 % im Falle der Trennung von Dimethylcarbonat und Methanol bedeuten, daß das hierbei gewonnene Methanol in einem weiteren Arbeitsgang von den restlichen 7 % an Dimethylcarbonat befreit werden muß.

Gemäß Beschreibung in EP-A-423 949 wird versucht, die beschriebenen Nachteile des Verfahrens nach EP-A-331 846 durch eine andere Membran zu überwinden, die ein Blend aus Polyvinylalkohol und Polyacrylsäure auf Polyacrylnitril als Stützmembran ist. Im Falle der Trennung von Dimethylcarbonat von Methanol wird eine Anreicherung von 73 % Methanol auf etwa 95 % bei einem Fluß von etwa 2 kg/m²·h erreicht; diese Anreicherung stellt auf der Retentatseite die Mindestanforderung für das gereinigte Dimethylcarbonat zur Verwendung in weiteren Prozessen dar.

Eine durch Plasmapolymerisation erhaltene Membran, die bevorzugt eine Composit-Membran darstellt, wird gemäß Beschreibung von EP-A-476 370 zur Abtrennung von Reaktionswasser aus einem Veresterungsgemisch eingesetzt. Ein Veresterungsgemisch gemäß dieser Beschreibung besteht aus nicht umgesetzter Carbonsäure, nicht umgesetztem Alkohol, dem als Reaktionsprodukt gewünschten Ester, dem dabei entstandenem Reaktionswasser und einem sauren Veresterungskatalysator. Der Abtrennung des Reaktionswassers an der Membran folgt sodann eine anderweitige Auftrennung des Retentats in den Ester-Produktstrom und in die zurückzuführenden, noch nicht umgesetzten Säuren und Alkohole. Das Verfahren von EP-A-476 370 ist demnach dadurch gekennzeichnet, daß außer dem Wasser keine wesentlichen organischen Bestandteile durch die Membran treten; insbesondere wichtig ist hierbei, daß der Veresterungsalkohol nicht durch die Membran tritt.

Die Plasmapolymerisation zur Modifikation von Oberflächen, beispielsweise von Membranen, ist bereits bekannt, beispielsweise aus Houben-Weyl, Methoden der organischen Chemie, Bd. VI/5b, Teil II (1975, S. 1.563 bis 1.591).

Es wurde ein Verfahren zur Abtrennung eines ersten Alkanols mit 1 bis 3 C-Atomen oder eines Gemisches dieses ersten Alkohols mit Wasser von sauerstoffhaltigen organischen Verbindungen mit 3 bis 7 C-Atomen, bestehend aus einem zweiten Alkanol und/oder Dialkylcarbonat, wobei das erste Alkanol stets mindestens 2 C-Atome weniger hat als jede der sauerstoffhaltigen organischen Verbindungen, oder zur Abtrennung von Wasser von aus Dialkylcarbonat bestehenden sauerstoffhaltigen organischen Verbindungen mit 3 bis 7 C-Atomen durch Pervaporation oder Dampfpermeation gefunden, wobei das beschriebene Gemisch bei 40 bis 130°C einer Membran zugeführt wird, die durch Plasmapolymerisation erhalten worden ist, wobei auf der Feed-Seite ein Druck von 0,5 bis 10 bar und auf der Permeatseite ein Druck von höchstens 100 mbar eingestellt wird und wobei als Permeat das erste Alkanol oder das Gemisch aus erstem Alkanol und Wasser oder Wasser selbst in angereicherter Form und als Retentat die sauerstoffhaltige organische Verbindung in angereicherter Form erhalten werden.

Vorzugsweise handelt es sich bei den zurückzuhaltenden Dialkylcarbonaten um symmetrische.

Das erfindungsgemäße Verfahren ist demnach beispielsweise für folgende Trennaufgaben geeignet: Die Abtrennung von Methanol oder Methanol/Wasser aus einem Gemisch mit den um mindestens 2 C-Atomen höheren Alkanolen, wie Propanol, Isopropanol, Butanol, Isobutanol, tert.-Butanol, den isomeren Pentanolen, Hexanolen und Heptanolen; die Abtrennung von Ethanol oder Ethanol/Wasser aus Butanol, Isobutanol, tert.-Butanol, den isomeren Pentanolen, Hexanolen und Heptanolen; die Abtrennung von Propanol oder Isopropanol oder deren Gemischen mit Wasser von den isomeren Pentanolen, Hexanolen und Heptanolen; die Abtrennung von Methanol oder Methanol/Wasser oder Wasser von Dimethylcarbonat; die Abtrennung von Ethanol oder Ethanol/ Wasser oder Wasser von Diethylcarbonat; die Abtrennung von Propanol beziehungsweise Isopropanol oder deren Gemischen mit H₂O oder Wasser selbst von Dipropylcarbonat beziehungsweise Diisopropylcarbonat.

Unter den angegebenen, durch das erfindungsgemäße Verfahren zu bewältigenden Trennaufgaben ist die Abtrennung des ersten Alkanols beziehungsweise seines Gemisches mit Wasser oder von Wasser von dem zugehörigen Dialkylcarbonat besonders wichtig. Ganz besonders wichtig ist die Aufgabe der Abtrennung von Methanol oder Methanol/H₂O oder von Wasser selbst von dem Gemisch mit Dimethylcarbonat (DMC). Im letzteren Falle hat das erste Alkanol Methanol genaue 2 C-Atome weniger als die sauerstoffhaltige organische Verbindung Dimethylcarbonat.

Das erfindungsgemäße Verfahren wird bei einer Feed-Temperatur von 40 bis 130°C, bevorzugt 40 bis 100°C durchgeführt. Auf der Feed-Seite wird ein Druck von 0,5 bis 10 bar, bevorzugt 0,8 bis 6 bar, besonders bevorzugt 1 bis 5 bar, eingestellt. Auf der Permeatseite wird ein Druck von höchstens 100 mbar, bevorzugt höchstens 20 mbar, beispielsweise 1 bis 100 mbar, bevorzugt 1 bis 20 mbar, eingestellt.

Bei der Durchführung des erfindungsgemäßen Verfahrens als Pervaporation wird das Feedgemisch in flüssiger Form an die Membran herangeführt, und der Druck und die Temperatur auf der Feed-Seite werden so eingestellt, daß der Druck oberhalb des Siededruckes des Feedgemischs liegt.

Es ist jedoch ebenfalls möglich, das erfindungsgemäße Verfahren in Form der Dampfpermeation durchzuführen, wobei das Feedgemisch dampfförmig oder in Form einer im Siedezustand befindlichen Flüssigkeit, bevorzugt dampfförmig, an die Membran gebracht wird. In diesem Falle werden Drücke und Temperaturen innerhalb der angegebenen Bereiche so gewählt und kombiniert, daß der Druck in Abhängigkeit von der Temperatur höchstens beim Dampfdruck des Feedgemischs, bevorzugt darunter, liegt. Gegebenenfalls kann im Fall der Dampfpermeation das dampfförmige Feedgemisch mit Hilfe eines Trägergasstromes an die Membran herangebracht werden, der aus einem inerten Gas oder einer Mischung mehrerer inerter Gase besteht. Brauchbare inerte Gase sind beispielsweise Stickstoff, die Edelgase, niedere Kohlenwasserstoffe, Luft, Kohlenmonoxid oder Kohlendioxid.

Das Permeat fällt zunächst dampfförmig an und kann in dieser Form von der Membran abgeführt oder vorher kondensiert und als Kondensat dem Permeatraum entnommen werden. Für den Fall einer gasförmigen Abführung kann auch hier ein inertes Trägergas der oben beschriebenen Art eingesetzt werden.

Der Aufbau einer Apparatur zur Durchführung des erfindungsgemäßen Verfahrens ist einfach und besteht aus einem Vorratsbehälter für das zu trennende Gemisch, einer Pumpe zur Einstellung des gewünschten Feed-Druckes, einem Modul mit der erfindungsgemäß einzusetzenden, durch Plasmapolymerisation erhältlichen Membran, einer Abführung des auf der Anströmseite der Membran zurückbleibenden Retentats und einer Abführungsmöglichkeit für das Permeat, wobei letztere vor allen Dingen durch die zur Aufrechterhaltung des Unterdruckes erforderliche Vakuumpumpe gekennzeichnet ist. Die Permeatseite kann vor oder hinter der Vakuumpumpe einen Kondensator aufweisen.

Es ist weiterhin möglich, das erfindungsgemäße Verfahren durch Behandlung einer vorbestimmten Gemischmenge quasi absatzweise zu betreiben oder es in vollkontinuierlicher Weise zu betreiben. Bei der absatzweisen Durchführung ist es weiterhin möglich, das Retentat in den Vorratsbehälter für das zu trennende Gemisch zurückzuführen und so die Gemischmenge wiederholt an die Membran zu führen, bis ein gewünschter Trenneffekt erreicht ist. Bei der vollkontinuierlichen Durchführung ist es weiterhin möglich, das Retentat an ein weiteres Modul zu bringen und somit eine mehrstufige Behandlung bis zur Erreichung des gewünschten Trenneffektes durchzuführen.

Erfindungsgemäß einzusetzende Membranen, die durch Plasmapolymerisation erhalten werden, zeichnen sich durch einen dichten porenfreien Aufbau aus. Zu ihrer Herstellung erzeugt man aus verschiedenen organischen Verbindungen durch die Einwirkung eines Plasma im Vakuum aktivierte Moleküle dieser organischen Verbindungen oder auch Bruchstücke davon, die auf einer gegenüberliegenden Elektrode niedergeschlagen werden und die dort bei der Abreaktion des reaktiven Zustandes polymerisieren. Zur Bildung des Plasmas benutzt man vielfach Glimmentladungen. Als Vakuum wird vielfach der Bereich von 10⁻³ bis 20 mbar angewandt. Aufgrund dieser Herstellungsweise finden sich in Plasmapolymeren keine sich wiederholenden Monomereinheiten, sondern es bildet sich ein dreidimensionales Netzwerk von unterschiedlichen Atomen und Atomgruppen.

Zur mechanisch sicheren Handhabung der durch Plasmapolymerisation entstandenen Membranen werden diese im allgemeinen auf ein poröses Trägermaterial aufgebracht. Dies kann ein Gewebe oder ein Vlies aus verschiedenen, bevorzugt temperaturbeständigen Polymeren sein. In weiterer Ausgestaltung kann anstelle eines solchen Trägermaterials oder zusätzlich zu einem Trägermaterial eine asymmetrische poröse erste Membran eingesetzt werden, auf der die porenfreie, dichte, durch Plasmapolymerisation hergestellte Membran in Form einer Composit-Membran niedergeschlagen wird, Asymmetrische poröse erste Membranen dieser Art werden beispielsweise durch die dem Fachmann bekannte Phaseninversionstechnik hergestellt. Die asymmetrische Membran wird so angeordnet, daß die Seite mit den größeren Poren die Rückseite bildet, die gegebenenfalls noch durch ein Gewebe oder Vlies der genannten Art abgeschlossen wird und daß die Seite mit den feineren Poren der Plasmabeschichtung zugewandt ist. Als Material für eine solche asymmetrische poröse Membran wird vielfach Polyacrylnitril eingesetzt.

Die organischen Verbindungen, die im Plasma aktiviert werden, können sehr verschiedenen Stoffklassen angehören. Genannt seien beispielsweise: gesättigte oder ungesättigte aliphatischen Kohlenwasserstoffe, wie Methan, Ethan, Propan, Butane, Hexane, Ethylen, Propylen, Butylen, Butadien, Acetylen; Halogenkohlenwasserstoffe auf der Basis der genannten aliphatischen Verbindungen mit Chlor-, Brom- und/oder Fluoratomen; aromatische Kohlenwasserstoffe, wie Benzol, Toluol, Xylol, Ethylbenzol; zur Polymerisation befähigte Monomere neben den obengenannten ungesättigten Kohlenwasserstoffen, wie Acrylnitril, Vinylchlorid, Acrylsäureester, Styrol und andere. Es ist auch bereits beschrieben worden (DE-A-3 730 065), aus kompakten Polymerkörpern im Plasma Bruchstücke in aktivierter Form herauszureißen und auf einer Gegenelektrode bzw. einer darauf angebrachten Stützschicht unter gleichzeitiger Desaktivierung wieder zu polymerisieren. Auch hierbei erhält man eine dichte porenfreie, durch Plasmapolymerisation hergestellte Membran.

Membranen aus Plasmapolymeren für Trennzwecke sind beispielsweise solche gemäß US-A-3 657 113. Das darin beschriebene Plasmapolymere wird bei einem Druck von 13,3-66,5 Pa (0,15 Torr) in einer Dicke von 0,03 bis 2 µm auf eine Schicht eines amorphen Polymeren aufgebracht. Als bevorzugte amorphe Polymere werden Polyphenylenoxid und Siloxane und ihre Copolymeren genannt. Als Plasmagas sind aromatische Verbindungen, Nitrile und mehrfach ungesättigte Verbindungen bevorzugt. Aus EP-A-0 134 055 ist weiterhin eine Komposit-Membran mit porenfreier Trennschicht bekannt, wobei die porenfreie, selektive Trennschicht mit einer Dicke von weniger als 0,1 µm auf einer dichten Polymerschicht aus einem herkömmlichen Polymeren aufgetragen ist. Die Schicht aus dem herkömmlichen Polymeren ist zwischen 0,01 und 5 µm dick, besteht vorzugsweise aus Polydimethylsiloxan und wird von einer porösen Unterstruktur getragen. Die durch Plasmapolymerisation gebildete selektive Trennschicht ist silikonfrei, kann ihrerseits aber auf der dem zu trennenden Gemisch zugewandten Seite noch eine dünne Schutzschicht tragen, welche bevorzugt aus dem gleichen Material besteht wie die Polymerschicht, auf die die Plasmaschicht aufgetragen ist, z.B. Silicon.

Bei den bevorzugt einzusetzenden, durch Plasmalpolymerisation hergestellten Membranen erfolgt die Abscheidung des die selektive Trennschicht bildenden Plasmapolymeren in einer Apparatur, die aus einem evakuierbaren Behälter mit Druckregelung und regelbaren Gaseinlaßsystemen sowie Einrichtungen zur Messung des Gesamtdruckes und der Partialdrucke der Gaskomponenten besteht. In dem Behälter befinden sich zwei einander gegenüberliegende Elektroden, deren eine geerdet ist. Auf einer Elektrode befindet sich das poröse Substrat, auf dem die Plasmapolymerschicht abgeschieden wird. Zwischen den beiden Elektroden liegt ein elektrisches Wechselfeld mit Frequenzen von 10 kHz bis 20 GHz mit regelbarer elektrischer Leistung des Wechselfeldes. In einer bevorzugten Ausführung wird hierbei eine Bahn des porösen Substrates mit geregelter Geschwindigkeit über eine Elektrode gezogen, so daß eine kontinuierliche Abscheidung einer Plasmapolymerschicht erfolgt. Es hat sich in überraschender Weise gezeigt, daß porenfreie, dichte Plasmapolymerschichten auf einem porösen Substrat direkt abgeschieden werden können, ohne daß zunächst eine dichte Zwischenschicht zwischen porösem Substrat und Plasmapolymerschicht aufgebracht werden muß. Nach dieser Verfahrensweise können poröse Materialien aus Kohlenstoff, Metall, Keramik oder Polymeren als Substrate benutzt werden, wobei bevorzugt Substrate in Form von porösen Membranen mit asymmetrischer Porenstruktur, z.B. aus Polyacrylnitril, Polysulfon oder anderen Polymeren Verwendung finden.

Wichtig ist dabei, daß diese Substrate auf der Oberfläche, auf der die Plasmapolymerschicht abgeschieden wird, Poren mit einem Durchmesser von weniger als 100 nm aufweisen. Größere Poren können mit der Plasmapolymerschicht nicht mehr sicher überbrückt werden, so daß Fehlstellen entstehen. Vorzugsweise besitzen die Poren eine möglichst einheitliche Porengröße mit einer mittleren Porenweite von 5 bis 40 nm. Poröse Membranen mit asymmetrischer Porenstruktur sind an sich bekannt und finden zum Beispiel in großem Umfang für die Ultrafiltration Verwendung. Üblicherweise besitzen sie eine Dicke von 30 bis 150 µm. Die kommerziell erhältlichen Membranen weisen zusätzlich einen Träger aus einem Vlies oder einem Gewebe auf und besitzen dann eine Gesamtdicke von 100 bis 300 µm.

Das im Bereich der Glimmentladung zwischen den Elektroden befindliche Gasgemisch, aus dem das Plasmapolymere gebildet wird, enthält mindestens eine matrixbildende und mindestens eine nicht-matrixbildende Kompotente sowie weiterhin eine sauerstoffhaltige Komponente.

Als matrixbildende Komponente können alle Kohlenwasserstoffe verwendet werden, die bei 50°C einen Dampfdruck von mindestens 0,5 mbar aufweisen. Beispiele für geeignete Kohlenwasserstoffe sind aliphatische Kohlenwasserstoffe bis 1 bis 12 C-Atomen, wie Methan, Butan oder Decan, oder aromatische Kohlenwasserstoffe, wie Benzol oder Toluol. Bevorzugt sind niedermolekulare Kohlenwasserstoffe mit mindestens einer C-C-Doppelbindung, wobei Ethen und Propen besonders bevorzugt sind.

Bei der nicht-matrixbildenden Komponente handelt es sich um anorganische Verbindungen, die Stickstoff, Silicium, Schwefel oder Phosphor enthalten und bei 50°C einen Dampfdruck von mindestens 1 mbar aufweisen. Beispiele für geeignete Verbindungen sind Wasserstoffverbindungen, wie Ammoniak, Silane, Schwefelwasserstoff oder Phosphine, oder Oxide von Stickstoff, Schwefel oder Phosphor, zum Beispiel Lachgas, Schwefeldioxid oder Schwefeltrioxid. Bevorzugt sind stickstoffhaltige Verbindungen, wie Ammoniak, Hydrazin oder Stickstoff, wobei Ammoniak besonders bevorzugt ist.

Geeignete sauerstoffhaltige Komponenten sind Sauerstoff, Kohlendioxid, Kohlenmonoxid und Wasser. Bevorzugt sind Sauerstoff, Kohlendioxid und Wasser.

Matrixbildende und nicht-matrixbildende Komponente werden im allgemeinen in einem Molverhältnis von 0,2 bis 5, bevorzugt 0,5 bis 1,5 eingesetzt.

Die sauerstoffhaltige Komponente wird im allgemeinen in einem Molverhältnis, bezogen auf die Summe aus matrixbildender und nichtmatrixbildender Komponente, von 0,05 bis 0,3, bevorzugt 0,1 bis 0,2, verwendet. Der Druck im Plasmareaktor beträgt im allgemeinen 10⁻³ bis 10 mbar, vorzugsweise 0,1 bis 1 mbar. Das elektrische Feld arbeitet im allgemeinen im Bereich von 10 kHz bis 5 GHz, vorzugsweise 20 kHz bis 14 MHz. Im allgemeinen wird mit einer elektrischen Leistung von 0,01 bis 3 Watt pro cm² Elektrodenfläche, vorzugsweise 0,1 bis 1 Watt pro cm² gearbeitet. Die Abscheidungszeit beträgt im allgemeinen 2 s bis 1 Stunde, vorzugsweise 20 s bis 10 min. Die Dicke der abgeschiedenen Plasmapolymerschicht beträgt im allgemeinen 0,1 bis 2 µm, vorzugsweise 0,5 bis 1 µm.

Es ist erstaunlich, daß für das angegebene Trennproblem in erfolgreicher Weise eine durch Plasmapolymerisation hergestellte Membran eingesetzt werden kann, von der aufgrund der bisher bekannt gewordenen Beschreibung anzunehmen war, daß auch niedere Alkohole auf der Retentatseite verbleiben.

### Beispiele

### Beispiel 1

Bei der pervaporativen Abtrennung von Methanol aus einem Dimethylcarbonat/Methanol-Gemisch wurden bei 50°C Feed-Temperatur, 3 bar Druck auf der Feed-Seite und 20 mbar Permeatdruck unter Einsatz der Membran Muster Nr. 270 von Fa. GFT, Neunkirchen-Heinitz (DE) folgende Versuchswerte erhalten (Tabelle 1):

**Tabelle 1**

| Feed-Konzentration Gew.-% Methanol | Permeatfluß kg/m²·h | Permeatkonzentration Gew.-% Methanol |
|---|---|---|
| 10 | 0,5 | 92 |
| 20 | 0,75 | 93,5 |
| 30 | 1,1 | 95,5 |
| 40 | 1,4 | 96,5 |
| 50 | 1,65 | 97 |
| 60 | 1,9 | 98 |
| 70 | 2,1 | 98 |

Bei 70°C Feed-Temperatur wurden etwa 2-fache Permeatflüsse erhalten.

### Beispiel 2

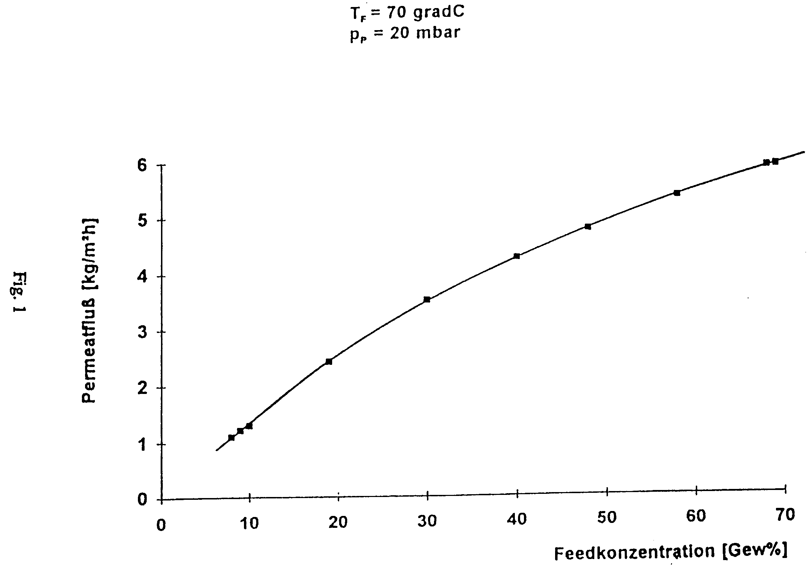

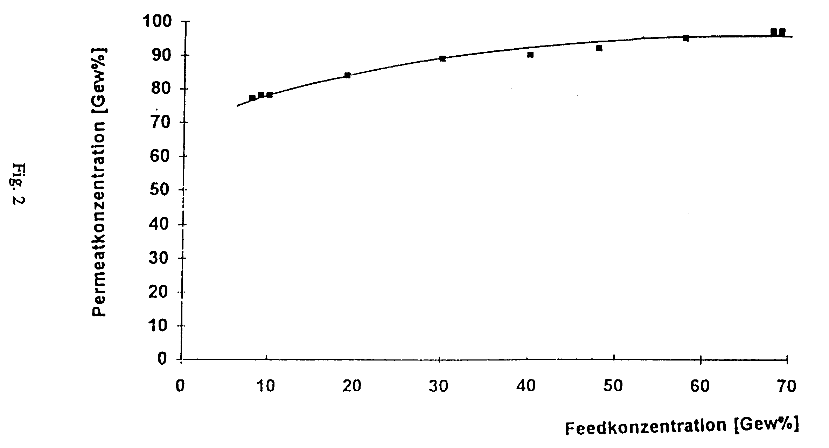

In zum Beispiel 1 analoger Weise wurden bei 70°C Feed-Temperatur und 20 mbar Permeatdruck unter Verwendung der Membran Muster Nr. 300 in Abhängigkeit von der Feed-Konzentration die in den anhängenden Fig. 1 und Fig. 2 gezeigten Permeatflüsse und Permeatkonzentrationen erhalten (System Dimethylcarbonat/Methanol).

### Beispiel 3

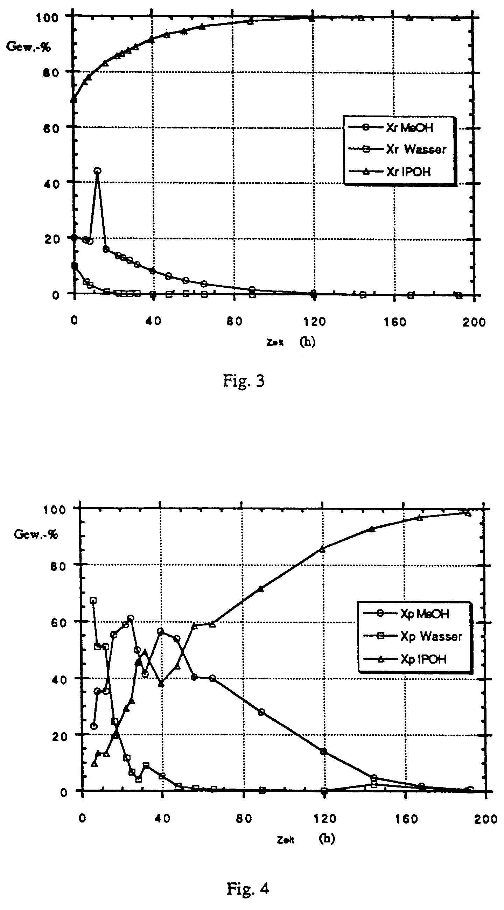

Ein Feedgemisch der Zusammensetzung 70 Gew.-% i-Propylalkohol (IPOH), 20 Gew.-% Methanol (MeOH) und 10 Gew.-% Wasser (H₂O) wurde an einer Membran Muster Nr. 229 bei 70°C Feed-Temperatur und 10 mbar Permeatdruck derart behandelt, daß das Permeat dem System entzogen wurde, aber das Retentat kontinuierlich als Feed recyclisiert wurde. In Abhängigkeit von der Zeit (h) erhielt man die in den anhängenden Fig. 3 und Fig. 4 gezeigten Zusammensetzungen von Retentat (Xr) bzw. Permeat (Xp).

### Beispiel 4

Bei der pervaporativen Abtrennung von Wasser aus einem Dimethylcarbonat/Wasser-Gemisch wurde unter Einsatz der Membran Muster Nr. 300 von Fa. GFT, Neunkirchen-Heinitz (DE) folgender Versuchswert erhalten:
Bei einem Wassergehalt im Feedgemisch von 2,7 Gew.-%, Rest DMC, wurde ein Permeatfluß von 0,8 kg/m²h und eine Permeatkonzentration von 94,7 Gew.-% Wasser ermittelt.

### Beispiel 5 (Herstellung erfindungsgemäß einsetzbarer Membranen; Prozentangaben sind in Gew.-%)

a) In einem evakuierbaren Gefäß, das mit einer Druckregelung, regelbaren Zuführungen für gas- und dampfförmige matrixbildende, nicht-matrix-bildende und sauerstoffhaltige Komponenten versehen war und zwei einander gegenüberliegende Elektroden enthielt, zwischen denen ein elektrisches Wechselfeld angelegt werden kann, wurde auf einer der Elektroden eine poröse Membran aus Polyacrylnitril mit asymmetrischer Porenstruktur angebracht. Die Membran besaß auf der "Feinseite" Poren mit einem mittleren Porendurchmesser von 20 nm. Das Gefäß wurde auf einen Gesamtdruck von 10⁻⁴ mbar evakuiert. Als matrixbildendes Gas wurde Ethylen mit einer Rate von 1,34 mmol/min. eingelassen, als nichtmatrixbildendes Gas Ammoniak mit 1,65 mmol/min. Der Gesamtdruck wurde auf 0,4 mbar gehalten. Zwischen den Elektroden, die jeweils eine Fläche von 630 cm² hatten, wurde eine elektrische Entladung mit einer Frequenz von 37 Kilohertz gezündet, wobei mit einer Leistung von 500 Watt gearbeitet wurde. Nach einer Abscheidungszeit von 5 min wurden Gaszufuhr und elektrisches Wechselfeld abgeschaltet, der Reaktor mit Luft geflutet und die Membran entnommen.
b) Wie unter a) beschrieben, wurde eine Plasmapolymermembran hergestellt. Die Gasflüsse der matrixbildenden und nicht-matrixbildenden Komponenten waren unverändert, ebenso der Gesamtdruck. Zusätzlich wurde Sauerstoff mit einer Rate von 0,38 mmol/min eingelassen; bei einer Frequenz von 37 kHz betug die elektrische Leistung 215 W, die Behandlungszeit 5 min. Man erhielt eine Trennschicht mit einer Dicke von 0,5 µm.
c) Eine Membran wie unter a) wurde hergestellt; als matrixbildendes Gas diente Ethen mit 1,27 mmol/min, als nicht-matrixbildendes Gas Ammoniak mit 1,7 mmol/min. Ferner wurde Kohlendioxid mit einer Rate von 0,22 mmol/min zugesetzt, der Gesamtdruck wurde auf 0,8 mbar eingestellt. Bei 37 kHz wurden 500 W 3 min lang aufrecht erhalten. Die erhaltene Membran besaß eine Trennschicht mit einer Dicke von 0,6 µm.
d) Wie unter a) wurde eine Membran hergestellt; als Substrat diente eine asymmetrische Polysulfon-Ultrafiltrationsmembran, die auf der Feinseite Poren mit einem mittleren Porendurchmesser von 30 nm besaß. Ethen wurde mit 1,34 mmol/min eingelassen, als nicht-matrixbildende Komponente Stickstoff mit 0,8 mmol/min, ferner Wasser mit 0,25 mmol/min. Bei einem Gesamtdruck von 0,8 mbar, einer Leistung von 425 W bei 37 kHz und einer Zeit von 5 min wurde eine Membran mit einer Trennschichtdicke von 0,5 µm erhalten.
e) Wie unter a) wurde eine Plasmapolymerschicht auf einer porösen asymmetrischen Polyacrylnitril-Ultrafiltrationsmembran mit einem mittleren Porenradius auf der Feinseite von 14 nm abgeschieden. Als matrixbildendes Gas diente Propen mit 1,3 mmol/min, als nicht-matrixbildende Komponente Ammoniak mit 1,6 mmol/min, ferner wurde 0,4 mmol/min Sauerstoff zudosiert. Bei einem Gesamtdruck von 0,8 mbar wurde mit 35 kHz bei 215 W 5 min behandelt. Hierbei wurde eine Membran mit einer Trennschicht von 0,5 µm Dicke erhalten.

## Patentansprüche

1. Verfahren zur Abtrennung eines ersten Alkanols mit 1 bis 3 C-Atomen oder eines Gemisches dieses ersten Alkanols mit Wasser von sauerstoffhaltigen organischen Verbindungen mit 3 bis 7 C-Atomen, bestehend aus einem zweiten Alkanol und/oder Dialkylcarbonat, wobei das erste Alkanol stets mindestens 2 C-Atome weniger hat als jede der sauerstoffhaltigen organischen Verbindungen oder zur Abtrennung von Wasser von aus Dialkylcarbonat bestehenden sauerstoffhaltigen organischen Verbindungen mit 3 bis 7 C-Atomen durch Pervaporation oder Dampfpermeation, wobei das beschriebene Gemisch bei 40 bis 130°C einer Membran zugeführt wird, die durch Plasmapolymerisation erhalten worden ist, wobei auf der Feed-Seite ein Druck von 0,5 bis 10 bar und auf der Permeatseite ein Druck von höchstens 100 mbar eingestellt wird und wobei als Permeat das erste Alkanol oder das Gemisch aus erstem Alkanol und Wasser oder Wasser selbst in angereicherter Form, und als Retentat die sauerstoffhaltige organische Verbindung in angereicherter Form erhalten werden.

2. Verfahren nach Anspruch 1, dadurch gekennzeichnet, daß das erste Alkanol oder sein Gemisch mit Wasser von symmetrischen Dialkylcarbonaten mit 3 bis 7 C-Atomen abgetrennt wird, wobei das erste Alkanol gleich dem Esteralkohol des Carbonats ist.

3. Verfahren nach Anspruch 2, dadurch gekennzeichnet, daß Methanol oder ein Methanol/Wasser-Gemisch von Dimethylcarbonat abgetrennt wird.

4. Verfahren nach Anspruch 1, dadurch gekennzeichnet, daß Wasser von symmetrischen Dialkylcarbonaten mit 3 bis 7 C-Atomen abgetrennt wird.

5. Verfahren nach Anspruch 1, dadurch gekennzeichnet, daß bei Durchführung als Pervaporation der Feed-Druck in Abhängigkeit von der Temperatur so eingestellt wird, daß er oberhalb des Siededrucks des Feeds liegt und daß bei Durchführung als Dampfpermeation der Feed-Druck in Abhängigkeit von der Temperatur so eingestellt wird, daß er höchstens beim Dampfdruck des Feeds, bevorzugt darunter, liegt.

6. Verfahren nach Anspruch 1, dadurch gekennzeichnet, daß eine Composit-Membran eingesetzt wird, die aus einem Trägermaterial und einer darauf durch Plasmapolymerisation aufgebrachten porenfreien Schicht besteht.

7. Verfahren nach Anspruch 6, dadurch gekennzeichnet, daß das Trägermaterial ein poröses Polymer ist, das auf seiner Feinseite Poren mit einem mittleren Porendurchmesser von unter 100 nm aufweist.

8. Verfahren nach Anspruch 6, dadurch gekennzeichnet, daß das Trägermaterial auf der Basis von Acrylnitril aufgebaut ist.

9. Verfahren nach Anspruch 7, dadurch gekennzeichnet, daß die Composit-Membran auf dem porösen Trägermaterial durch Plasmapolymerisation eines oder mehrerer Kohlenwasserstoffe als matrixbildender Komponente und einer oder mehrerer Stickstoff, Silicium, Schwefel oder Phosphor enthaltender anorganischer Verbindungen als nicht-matrixbildender Komponente mit Hilfe einer Glimmentladung in einem elektrischen Wechselfeld hergestellt wird, wobei in Abwesenheit oder Anwesenheit einer sauerstoffhaltigen Verbindung gearbeitet wird.

10. Verfahren nach Anspruch 9, dadurch gekennzeichnet, daß als matrixbildende Komponente Ethen oder Propen, als nicht-matrixbildende Komponente Ammoniak oder Stickstoff und als sauerstoffhaltige Verbindung Sauerstoff, Kohlendioxid oder Wasser eingesetzt werden.

## Claims

1. Process for separating off a first alkanol having 1 to 3 C atoms, or a mixture of this first alkanol with water, from oxygen-containing organic compounds having 3 to 7 C atoms and consisting of a second alkanol and/or dialkyl carbonate, the first alkanol always having at least 2 C atoms less than each of the oxygen-containing organic compounds, or for separating off water from oxygen-containing organic compounds which have 3 to 7 C atoms and consist of dialkyl carbonate, by pervaporation or vapour permeation, the mixture described being fed at 40 to 130°C to a membrane which has been obtained by plasma polymerization, a pressure of 0.5 to 10 bar being established on the feed side and a pressure of not more than 100 mbar being established on the permeate side, and the first alkanol or the mixture of first alkanol and water, or water itself in concentrated form being obtained as the permeate, and the oxygen-containing organic compound in concentrated form being obtained as the retained material.

2. Process according to Claim 1, characterized in that the first alkanol or its mixture with water is separated off from symmetric dialkyl carbonates having 3 to 7 C atoms, the first alkanol being the same as the ester-alcohol of the carbonate.

3. Process according to Claim 2, characterized in that methanol or a methanol/water mixture is separated off from dimethyl carbonate.

4. Process according to Claim 1, characterized in that water is separated off from symmetric dialkyl carbonates having 3 to 7 C atoms.

5. Process according to Claim 1, characterized in that when carried out as pervaporation, the feed pressure is established as a function of the temperature such that it is above the boiling pressure of the feed, and in that when carried out as vapour permeation, the feed pressure is established as a function of the temperature such that it is not more than the vapour pressure of the feed, and is preferably below it.

6. Process according to Claim 1, characterized in that a composite membrane which comprises a carrier material and a pore-free layer applied thereto by plasma polymerization is employed.

7. Process according to Claim 6, characterized in that the carrier material is a porous polymer which has on its fine side pores having an average pore diameter of less than 100 nm.

8. Process according to Claim 6, characterized in that the carrier material is built up on the basis of acrylonitrile.

9. Process according to Claim 7, characterized in that the composite membrane on the porous carrier material is prepared by plasma polymerization of one or more hydrocarbons as the matrix-forming component and of one or more nitrogen-, silicon-, sulphur- or phosphorus-containing inorganic compounds as the component which is not matrix-forming, with the aid of a glow discharge in an electrical alternating field, the preparation being carried out in the absence or presence of an oxygen-containing compound.

10. Process according to Claim 9, characterized in that ethene or propene is employed as the matrix-forming component, ammonia or nitrogen is employed as the component which is not matrix-forming and oxygen, carbon dioxide or water is employed as the oxygen-containing compound.

## Revendications

1. Procédé pour la séparation d'un premier alcanol contenant de 1 à 3 atomes de carbone ou d'un mélange de ce premier alcanol avec de l'eau, de composés organiques oxygénés contenant de 3 à 7 atomes de carbone, constitués par un deuxième alcanol et/ou par du dialkylcarbonate, le premier alcanol contenant toujours au moins deux atomes de carbone de moins que celui des composés organiques oxygénés, ou bien pour la séparation d'eau de composés organiques oxygénés constitués par du dialkylcarbonate, contenant de 3 à 7 atomes de carbone, par pervaporation ou par perméation à la vapeur, le mélange décrit étant acheminé à une membrane à une température de 40 à 130°C, que l'on a obtenu par polymérisation au plasma, dans lequel on règle, côté alimentation, une pression de 0,5 à 10 bar et, côté perméat, une pression maximale de 100 mbar, et dans lequel on obtient, comme perméat, le premier alcanol ou le mélange constitué par le premier alcanol et de l'eau ou encore de l'eau elle-même, sous forme enrichie, et comme rétentat, le composé organique oxygéné sous forme enrichie.

2. Procédé selon la revendication 1, caractérisé en ce que le premier alcanol ou son mélange avec de l'eau est séparé de dialkylcarbonates symétriques contenant de 3 à 7 atomes de carbone, le premier alcanol étant égal à l'ester-alcool du carbonate.

3. Procédé selon la revendication 2, caractérisé en ce qu'on sépare du méthanol ou un mélange de méthanol/eau de diméthylcarbonate.

4. Procédé selon la revendication 1, caractérisé en ce qu'on sépare de l'eau de dialkylcarbonates symétriques contenant de 3 à 7 atomes de carbone.

5. Procédé selon la revendication 1, caractérisé en ce que, lorsqu'on procède à la pervaporation, on règle la pression d'alimentation en fonction de la température de telle sorte qu'elle se situe au-dessus de la pression d'ébullition du produit d'alimentation et en ce que, lorsqu'on procède à la perméation à la vapeur, on règle la pression d'alimentation en fonction de la température de telle sorte qu'elle se situe au maximum à la pression de vapeur du produit d'alimentation, de préférence en dessous.

6. Procédé selon la revendication 1, caractérisé en ce qu'on met en oeuvre une membrane composite, qui est constituée par une matière de support, et par une couche exempte de pores appliquée sur la première citée par polymérisation au plasma.

7. Procédé selon la revendication 6, caractérise en ce que la matière de support est un polymère poreux qui présente, sur son côté fin, des pores possédant un diamètre moyen inférieur à 100 nm.

8. Procédé selon la revendication 6, caractérisé en ce que la base de la composition de la matière de support est de l'acrylonitrile.

9. Procédé selon la revendication 7, caractérisé en ce que la membrane composite est préparée sur la matière de support poreuse par polymérisation au plasma d'un ou de plusieurs hydrocarbures comme composant formateur de matrice et d'un ou de plusieurs composés inorganiques contenant de l'azote, du silicium, du soufre ou du phosphore, comme composant non formateur de matrice, à l'aide d'un effluve dans un champ alternatif électrique, en travaillant en l'absence ou en présence d'un composé oxygéné.

10. Procédé selon la revendication 9, caractérisé en ce qu'on met en oeuvre, comme composant formateur de matrice, l'éthène ou le propène; comme composant non formateur de matrice, l'ammoniac ou l'azote; et comme composé oxygéné, l'oxygène, le dioxyde de carbone ou l'eau.
